# EUROPEAN PATENT APPLICATION

(11) **EP 0 919 243 A1**
(43) Date of publication of application: **02.06.1999**
(21) Application number: 97203671.9
(22) Date of filing: 25.11.1997
(51) Int. Cl.: A61K 39/39, C07K 14/245

(54) **Vaccine containing B subunits of heat-labile enterotoxin (LTB) of Escherichia coli as an adjuvant**

(71) Applicant: DUPHAR INTERNATIONAL RESEARCH B.V, 1381 CP Weesp (NL); Universiteit van Groningen, Groningen (NL)
(72) Inventor: The designation of the inventor has not yet been filed
(74) Representative: Breepoel, Peter M.

(57) **Abstract**

The present invention relates to a vaccine containing at least one immunogen and an adjuvanting amount of B subunits of heat-labile enterotoxin characteristic of *E. coli* isolated from a non-human source.
This vaccine is particularly suitable for induction of a protective response against said immunogen upon mucosal (e.g. intra-nasal) administration.

## Description

The present invention relates to a vaccine containing the B subunits of heat-labile enterotoxin (LTB) of *Escherichia coli* (*E. coli*) as a mucosal immunoadjuvant. The invention relates in particular to a vaccine of this type to prevent influenza infections in humans. However, the invention is not restricted to application in influenza vaccines.

It is the object of vaccination against infectious diseases to prevent or at least restrain infection of the vaccinated subject by stimulating an immune response against the infectious agent through introduction of an antigen formulation derived from the particular pathogen. Ideally, the induced immune response should consist of two components, a humoral response (the production of antigen-specific antibodies) and a cellular response (the generation of specific cytotoxic T lymphocytes, capable of eliminating cells infected by the pathogen).

Many vaccination procedures involve the administration of a formulation containing inactivated or attenuated whole pathogen. However, there is a considerable disadvantage to vaccination with whole pathogen, since such preparations, even though they are usually highly immunogenic, may have undesirable side effects. This explains the current trend towards the use of well-defined subunit or synthetic vaccines, substantially lacking the adverse side effects of the whole infectious agent. However, compared to whole pathogen, subunit or synthetic vaccines are often not very immunogenic, at least in the absence of an added adjuvant.

Adjuvants are substances or materials administered in conjunction with the antigen so as to stimulate the immune response against that antigen. There is a need for appropriate adjuvants which would boost the immune response against subunit or synthetic antigens without causing undesirable side effects.

Influenza vaccine formulations have contained for a long time, and in some cases still contain, inactivated or attenuated whole virus. Such formulation may have considerable side effects, most notably fever and reactions at the site of injection. Nowadays, vaccination is usually done with a subunit formulation. This subunit vaccine, which causes less side reactions, only contains the two major surface antigens of the virus, the hemagglutinin (HA) and the neuraminidase (NA), in a more or less purified form. In most current vaccine formulations there is no added adjuvant present.

The inactivated or attenuated whole influenza virus vaccine as well as the subunit vaccine are usually administered via a single intramuscular (i.m.) injection. The protection against influenza infection, achieved by either vaccination procedure, is comparatively low, particularly in elderly people. The relatively low efficacy of vaccination against influenza is due in part to the high antigenic variability of the virus. However, there is reason to believe that the protection against influenza infection by vaccination can be improved by stimulation and/or modification of the immune response against the vaccine.

In the case of influenza, or in general in cases in which the infection is contracted via the respiratory tract, strategies for improved vaccination efficacy should aim at the generation of not only an adequate T-cell-dependent IgG response in the circulation, but also at a local immune response (secretory IgA) in the lungs and nasal cavity as a first line of defence against invading infectious virus. Furthermore, a cellular immune response (cytotoxic T-cells) might also be important, particularly in restricting the infection. It has been demonstrated that administration of influenza vaccine via i.m. injection (the current route of administration) does not result in a local IgA response in the respiratory tract.

The present invention relates to the surprising finding that the presence of LTB in an intranasal vaccine formulation not only stimulates the IgG response in the circulation, relative to i.m. immunisation with the adjuvant-free immunogen vaccine, but also generates a local IgA response in the respiratory tract.

The intact heat-labile enterotoxin (LT) is composed of one A subunit and a pentamer of five B subunits. The former subunit attributes the toxic activity to LT and hence the presence of A subunit or LT holotoxin in a vaccine is undesirable.

The use of LTB as an adjuvant for in particular influenza antigens has been investigated by Tamura and co-workers (Hirabashi et al.: Vaccine **8**: 243-248 [1990]; Kikuta et al.,: Vaccine **8**: 595-599 [1990]; Tamura et al. J.: Immunology **3**: 981-988 [1992]; Tamura et al.: Vaccine **12**: 419-426 [1994]; Tamura et al.: Vaccine **12**: 1083-1089 [1994]). By these studies it was established that the presence of trace amounts of holotoxin is essential for adjuvant activity of the LTB used. The source of this heat-labile enterotoxin (LT) was *E. coli* isolated from humans.

Surprisingly, it was found that vaccines containing an immunogen of an infective agent (e.g. virus, bacteria) and LTB derived from the heat-labile enterotoxin characteristic of *E. coli* not isolated from humans, but from pigs induce considerable antibody titres against the antigen and provide protection of immunised mice against infection with said agent.

Accordingly, the present invention is concerned with a vaccine containing at least one immunogen and an adjuvanting amount of the B subunit of heat-labile enterotoxin characteristic of *E. coli* isolated from a non-human source, and free of toxic A subunits or toxic LT holotoxin.In particular LTB prepared by recombinant DNA techniques can be utilised.

Such vaccines were found not only to induce systemic immunoglobulin (e.g.IgG) against the immunogen upon mucosal (e.g. intra nasal) administration, but also to induce local secretion of IgA.

This latter property is particularly favourable for immunisation against diseases which are transmitted by mucosal infection with viruses (such as influenza virus, herpes virus, papilloma virus) or bacteria (like chlamydia, pneumococs), or fungi.

A particular advantage of mucosal administration is the ease of vaccine application, which, furthermore, circumvents potential needlefobia with vaccinees receiving an intramuscular immunisation.

Although, for example in the case of influenza infection, high serum IgG titres are important for preventing systemic spread of the virus and protection of the lungs against infection, local S-IgA antibodies are crucial as a first line of defence for protection of the upper respiratory tract.

It has been reported that mucosal vaccination by i.n. administration of inactivated influenza virus in the absence of a mucosal adjuvant was not successful (Clancy: Drugs **50**: 587-594 [1995]; Katz et al.; J. Infect. Dis. **175**: 352-369 [1997]), probably because direct administration of an antigen to mucosal tissue will not result in an S-IgA response. Co-administration of a mucosal adjuvant seems to be a prerequisite to induce a local immune response against an immunogen. Remarkably, it was found that by i.n. immunisation according to the present invention the so-called common mucosal immune system is activated which results in secretion of S-IgA not only at the site op application (i.n.) but also in distant mucosal tissues.

Vaccines according to the present invention may contain immunogens of e.g. viral or bacterial origin, such as bacterial antigens, viral subunits (optionally inactivated) split viruses (optionally inactivated) inactivated viruses or bacteria, or attenuated (e.g. cold-adapted) live viruses.

The LTB used according to the present invention may be purified LTB from a natural source which is free of toxic LTA, or recombinant LTB, or part of the LTB prepared by recombinant techniques. Free of toxic LTA in the present context means strictly LTA-free.

In the vaccine according to the present invention the LTB can be used freely admixed with the antigen - a covalent coupling between the antigen and the adjuvant is not needed to attain adequate adjuvant effect.

Apart from LTB and one or more immunogens the vaccine according to the present invention may contain an aqueous solvent, in particular a buffer, more in particular PBS (phosphate-buffered saline) as well as a stabiliser (e.g. PEG or methyl cellulose), and or glucose.

The components of the vaccine according to the present invention may be freeze dried or in a liquid form.

The vaccine according to the present invention may be present e.g. in bulk, or in an ampoule, or in a syringe, or in a nebuliser.

The vaccine according to the present invention may be administered by subcutaneous, or intramuscular, or intra-bronchial, or intra-nasal or intra-vaginal application or per os.

### EXAMPLE 1

### PREPARATION OF RECOMBINANT LTB AND INFLUENZA SUBUNIT ANTIGEN

### RECOMBINANT LTB

The pROFIT expression vector, which contains a temperature inducible λPR promoter was used for cloning (van der Linden et al.: Eur. J. Biochem. **204**: 197-202 [1992]).
Construction of the plasmid encoding the B subunit of LT (pROFIT-LTB) was carried out according to the method of De Haan et al. (Vaccine **14**: 260-266 [1996]).
*E. coli* MC1061 was used as a host strain for the pROFIT plasmid construct.
Bacteria were grown on Luria-Bertani medium containing 50 µg of kanamycin per ml.
Induction of LTB expression and purification of the protein were carried out as described by De Haan et al. (*supra*).
Recombinant proteins were purified using immobilised D-galactose (Pierce) as previously described by Uesaka et al. (Microb. Path. **16**: 71-76 [1994]). Column fractions containing purified protein were pooled, dialysed against PBS and stored at 4°C.

### INFLUENZA SUBUNIT ANTIGEN

The influenza subunit antigen was prepared from B/Harbin/7/94 virus (B/Harbin) or A/Johannesburg/33/94 (A/Johannesburg) grown on embryonated chicken eggs according to the method described by Chaloupka et al. (Eur. J. Microbiol. Infect. Dis. **15**: 121-127 [1996]).
The potency of the subunit antigen preparations, expressed as µg per ml, was determined in a single-radial diffusion test according to Wood et al. (J. Biol. Stand. **5**: 237-241 [1977]).

### EXAMPLE 2

### SYSTEMIC ANTIBODY RESPONSE TO INFLUENZA SUBUNIT VACCINE

Groups of four mice were immunised i.n. without anaesthesia with 5 µg of influenza subunit antigen derived from either B/Harbin or A/Johannesburg virus. The antigen was given either alone or together with 2.0 µg of LTB, in all cases in a volume of 20 µl on days 0, 7 and 14. Control mice received the same volume of PBS. Mice were sacrificed on day 28. Serum IgG antibody response was determined in a direct ELISA.

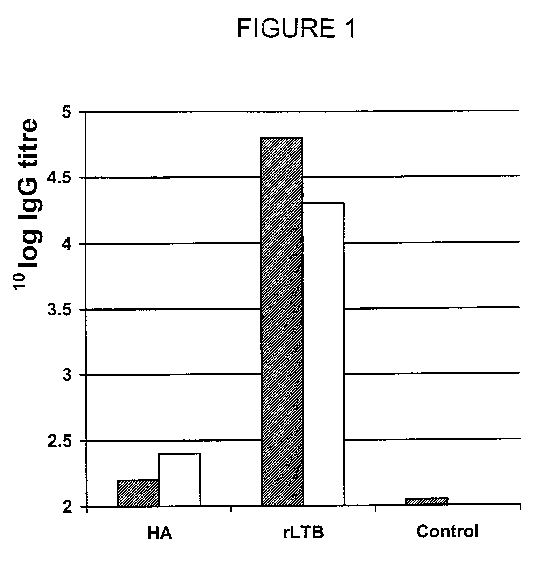

Figure 1 shows the observed serum IgG antibody responses against HA B/Harbin (solid bars) and HA A/Johannesburg (open bars).
Administration of the subunit antigen without adjuvant gave poor asystemic antibody response, whereas supplementation of the subunit antigen with rLTB enhanced the serum antibody response by more than two orders of magnitude. Differences between the responses of mice immunised with B/Harbin and A/Johannesburg were not significant.
These results show that non-toxic rLTB is a powerful adjuvant capable of inducing high systemic antibody responses towards i.n. administered influenza subunit antigen.

### EXAMPLE 3

### INDUCTION OF LOCAL MUCOSAL ANTIBODY RESPONSE TO INFLUENZA SUBUNIT VACCINE

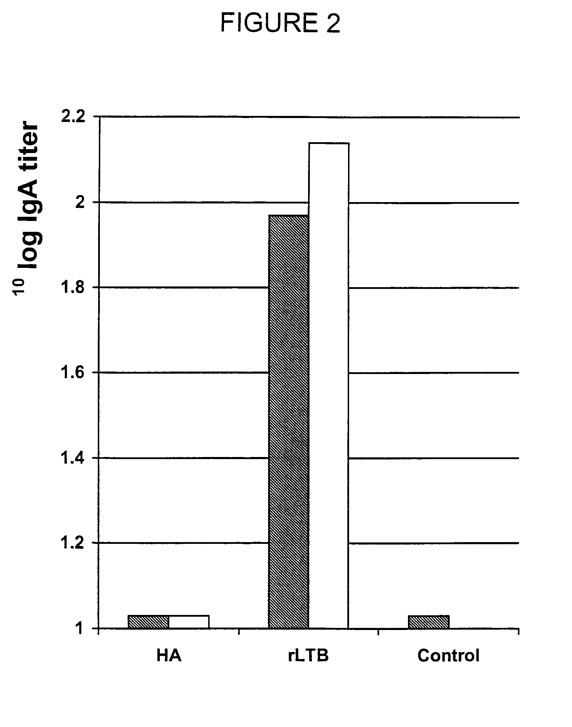

In order to investigate the ability of rLTB to evoke influenza HA-specific S-IgA responses, nasal washes of the mice from EXAMPLE 2 were analysed for the presence of influenza -specific IgA antibodies.
The results are shown in Figure 2.
The data show that rLTB induced local S-IgA responses against HA. The two different influenza subunit antigens gave similar results.

### EXAMPLE 4

### INDUCTION OF GENITAL MUCOSAL ANTIBODY RESPONSE TO INFLUENZA SUBUNIT VACCINE APPLIED I.N.

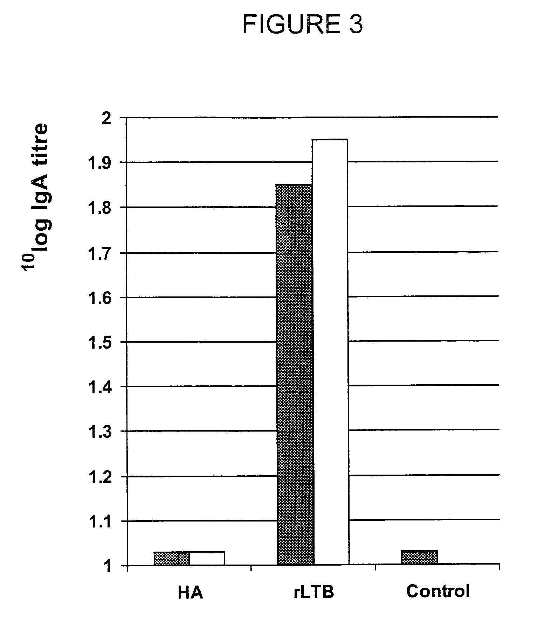

In order to investigate the ability of rLTB to evoke influenza HA-specific S-IgA responses at mucosal sites other than the site of administration, the induction of influenza-specific S-IgA antibodies in the genital tract after i.n. immunisation in the mice from EXAMPLE 2 was investigated.
The results are shown in Figure 3.
These results show that rLTB proved effective in inducing S-IgA responses at this distant mucosal site. Both B/Harbin and A/Johannesburg antigen responded equally well.

### EXAMPLE 5

### KINETICS OF IGG RESPONSE

Four groups of eight female BALB/c mice (6-8 weeks) each were treated as follows
- Control: treated with PBS without antigen. 20 µl i.n. without anaesthesia on days 0, 7 and 14
- rLTB: 5 µg HA and 2.0 µg recombinant LTB in 20 µl applied i.n. without anaesthesia on days 0, 7 and 14
- HA s.c.: 5 µg HA in 100 µl applied s.c. without anaesthesia on day 0
- Conv.: infected with 10⁸ infective units of PR8 virus, in 20 µl applied i.n. without anaesthesia on day 0

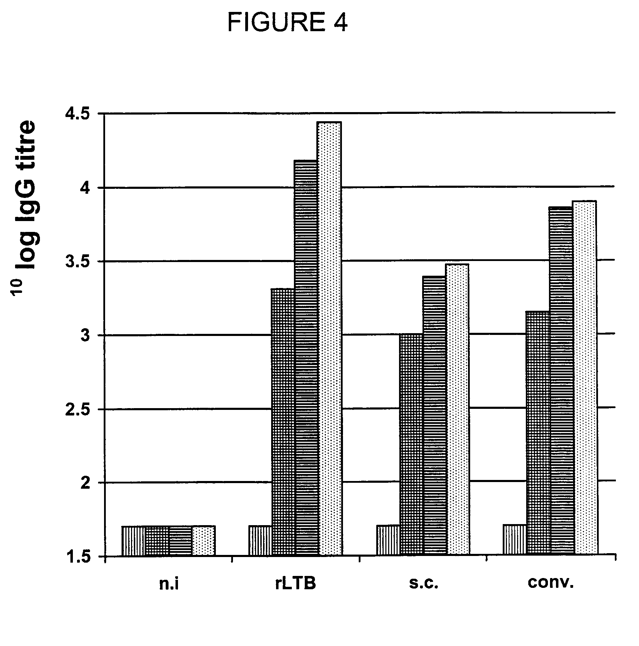

From four mice of each group blood samples were taken from the tail veins on day 6, 13 and 20. Furthermore , on day 28 all mice were sacrificed and bled. In each sample serum IgG was measured by ELISA.
The results are shown in Figure 4. The bars (from left to right) for each of the vaccination regimens represent the IgG titres on days 6, 13, 20 and 28, respectively.

These results show that after i.n. vaccination with HA/rLTB the IgG induction is of at least the same magnitude as after s.c. vaccination with HA alone, or as in convalescent mice.

### EXAMPLE 6

### NASAL AND LUNG MUCOSAL ANTIBODIES

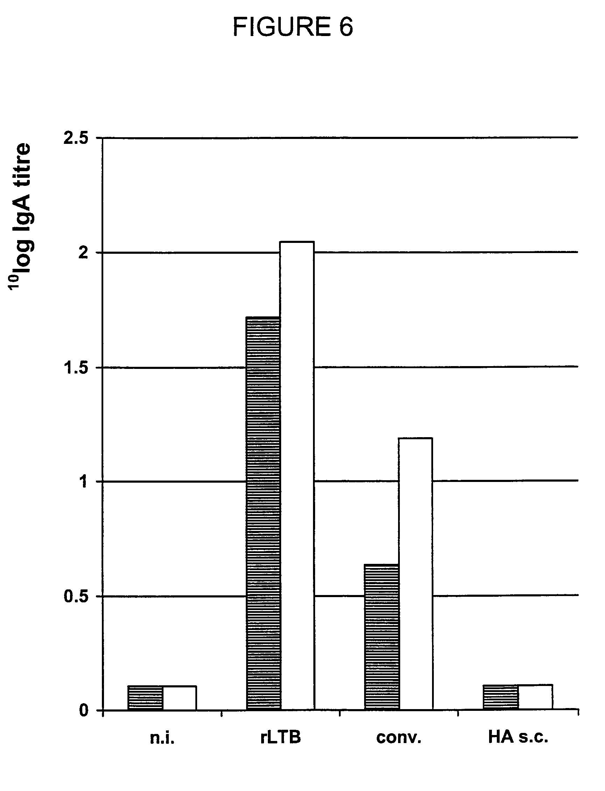

The same mice which were studied in EXAMPLE 5 underwent mucosal lavages of the nasal cavity and the urogenital tract after being sacrificed on day 28. Nasal lavage fluids were collected by flushing 0.5 ml of PBS retrograde via the nasopharynx to the upper part of the trachea, flushing back and collecting the lavage fluid at the nostrils.
Lavages of the urogenital tract were conducted by introducing and withdrawing a 100 µl volume of PBS ten times into the vagina using a pipette tip. Mucosal washes were stored at 4 °C until determination of their IgA content by ELISA.
The results are summarised in Figure 6. The hatched bars represent the data from nasal washes and the open bars show the data from vaginal washes.
The results indicate that the titre of the first line of defence antibodies (S-IgA) upon in. vaccination with HA/rLTB is of at least the same magnitude as the S-IgA titre in convalescent mice whereas the (classical) s.c. vaccination with HA does not lead to a substantial mucosal IgA titre.

### EXAMPLE 7

### PROTECTION OF VACCINATED MICE AGAINST CHALLENGE

Four mice of each of the groups of EXAMPLE 5 were infected on day 28 with 5x10⁶ infective units of PR8 virus i.n. in 20 µl without anaesthesia.
Three days post-challenge virus load was determined in nose and lungs.

Virus titration in nose and lung homogenates was carried out on MDCK cells which were cultured on EPISERF (Life Technologies, PAISLY, Scotland) in microtitration plates by two-step dilutions, and by subsequent endpoint determination using haemagglutination with guinea pig erythocytes.

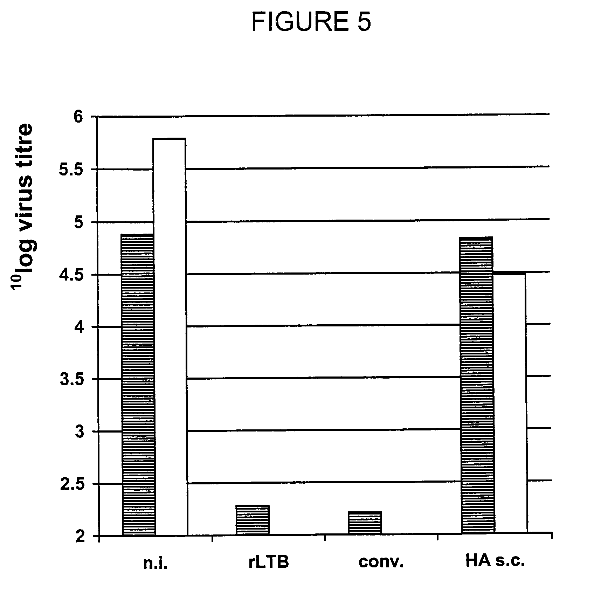

The results are summarised in Figure 5. The hatched bars represent the virus titres in the nose and the open bars are for the lungs. The virus titres in the lungs for convalescent mice and upon vaccination with rLTB were insignificant. Hence, these data show that by using rLTB as a mucosal adjuvant, protection against influenza infection is adequately enhanced.

## Claims

1. Vaccine containing at least one immunogen and an adjuvanting amount of B subunits of heat-labile enterotoxin (LTB) characteristic of *E. coli* isolated from a non-human source.

2. Vaccine according to claim 1, wherein the LTB is free from contaminating A subunits or holotoxin.

3. Vaccine according to claim 1-2, wherein the LTB is prepared by recombinant DNA methods.

4. Vaccine according to claim 1-3, wherein viral or bacterial or fungal antigens are used as an immunogen.

5. Vaccine according to claim 1-3, wherein the immunogen provides immunisation against a disease which is transmitted by mucosal infection.

6. Vaccine according to claim 5, wherein influenza antigens are used as an immunogen.

7. Method for the induction of a systemic immunoglobulin response against an immunogen by mucosal administration of said immunogen and an adjuvanting amount of B subunits of heat-labile enterotoxin characteristic of *E. coli* isolated from a non-human source.

8. Method for the induction of a common mucosal immune response against an immunogen by mucosal administration of said immunogen and an adjuvanting amount of B subunits of heat-labile enterotoxin characteristic of *E. coli* isolated from a non-human source.

9. Use of the B subunits of heat-labile enterotoxin (LTB) characteristic of *E. coli* isolated from a non-human source in the preparation of a vaccine comprising an immunogen and an adjuvanting amount of said LTB suitable for the induction of a systemic immunoglobulin response against said immunogen in an individual upon mucosal administration.

10. Use of the B subunits of heat-labile enterotoxin (LTB) characteristic of *E. coli* isolated from a non-human source in the preparation of a vaccine comprising an immunogen and an adjuvanting amount of said LTB suitable for the induction of a common mucosal immune response against said immunogen in an individual upon local mucosal administration.
